(19) Europäisches Patentamt
European Patent Office
Office européen des brevets

(11) Numéro de publication : **0 376 850 B1**

# (12) FASCICULE DE BREVET EUROPEEN

(45) Date de publication du fascicule du brevet : **03.03.93 Bulletin 93/09**

(21) Numéro de dépôt : **89403670.6**

(22) Date de dépôt : **28.12.89**

(51) Int. Cl.⁵ : **C07C 211/27,** C07C 211/28, C07C 211/29, C07C 215/30, C07C 225/14, A61K 31/35

## (54) Dérivés du benzène, leur préparation et compositions pharmaceutiques les contenant.

(30) Priorité : **30.12.88 FR 8817538**

(43) Date de publication de la demande : **04.07.90 Bulletin 90/27**

(45) Mention de la délivrance du brevet : **03.03.93 Bulletin 93/09**

(84) Etats contractants désignés : **AT BE CH DE ES FR GB GR IT LI LU NL SE**

(56) Documents cités :
**EP-A- 0 040 744**
**EP-A- 0 224 163**
**DE-A- 2 451 474**
**FR-A- 1 468 761**
**FR-A- 2 117 942**
**FR-A- 2 455 889**
**JOURNAL OF MEDICINAL CHEMISTRY, vol. 11, no. 1, Janvier 1968, pages 95-97; J. MILLS et al.: "N-Substituted Cyclopropylamines as Monoamine Oxidase Inhibitors. Structure-Activity Relationships. Dopa Potentiation in Mice and in Vitro Inhibition of Kynuramine Oxidation"**

(73) Titulaire : **ELF SANOFI**
**32-34, rue Marbeuf**
**F-75008 Paris (FR)**

(72) Inventeur : **Brelière, Jean-Claude**
**617, Rue Antoine-Laurent Jussieu**
**F-34090 Montpellier (FR)**
Inventeur : **Casellas, Pierre**
**10, Rue Carl Von Liné**
**F-34000 Montpellier (FR)**
Inventeur : **Lavastre, Serge**
**L'Hortus 58 A2 541 Grand Mail**
**F-34080 Montpellier (FR)**
Inventeur : **Paul, Raymond**
**75, Rue des Chanterelles**
**F-34980 St Gely du Fesc (FR)**

(74) Mandataire : **Gillard, Marie-Louise et al**
**Cabinet Beau de Loménie 55, Rue d'Amsterdam**
**F-75008 Paris (FR)**

EP 0 376 850 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés du benzène présentant une activité sur le système immunitaire. Elle a également pour objet leur préparation et les compositions pharmaceutiques les contenant.

Le brevet français 2 249 659 décrit des composés de formule :

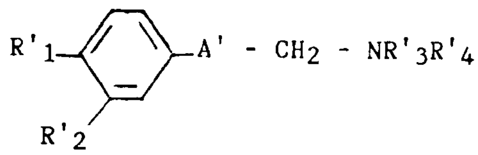

1

dans laquelle A′ représente le groupe $-CH_2-CH_2-$ ou $-CH = CH-$ ;

- $R'_1$ représente un cyclohexyle ou un phényle ;
- $R'_2$ représente l'hydrogène ou un halogène ;
- $R'_3$ représente un atome d'hydrogène ou un groupe alkyle en $C_1-C_3$ ;
- $R'_4$ représente un groupe alkyle en $C_1-C_3$ ;

ou $R'_3$ et $R'_4$ considérés ensemble avec l'atome d'azote auquel ils sont liés peuvent former un groupe hétérocyclique.

Selon ce document, les composés 1 présentent des propriétés psychostimulantes.

La demande de brevet européen 224 163 décrit des composés de formule :

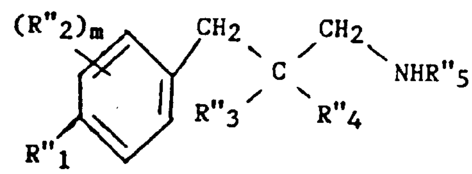

2

dans laquelle les divers substituants peuvent avoir les significations suivantes :

$R''_1$ et $R''_2$ représentent un hydrogène, un alkyle, un cycloalkyle ou un halogène ;

$R''_3$ et $R''_4$ sont l'hydrogène ou un alkyle ;

$R''_5$ est un alkyle ou un cycloalkyle.

La plupart des composés exemplifiés dans cette demande de brevet répondent à la formule 2 dans laquelle $R''_1$ = tert-$C_4H_9$, $R''_3$ = H et $R''_4$ = $CH_3$.

Cette demande de brevet ne décrit aucun composé ayant pour substituant $R''_3$ et $R''_4$ = H et/ou $R''_2$ = cycloalkyle ou phényle.

D'après la description de ce brevet, les composés décrits présentent une activité fongicide sur les champignons pathogènes des plantes.

Selon la présente invention, il a maintenant été trouvé une nouvelle famille de dérivés du benzène qui possèdent des propriétés inattendues : en effet, ces composés présentent d'intéressantes propriétés sur le système immunitaire.

La présente invention a pour objet des composés de formule :

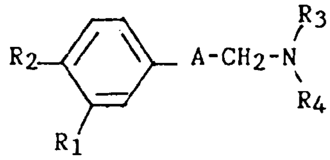

(I)

dans laquelle :

- $R_1$ représente un atome d'hydrogène ou un atome d'halogène ;
- $R_2$ représente un cyclohéxyle ou un phényle ;
- $R_3$ représente un cycloalkyle contenant de 3 à 6 atomes de carbone ;
- $R_4$ représente un atome d'hydrogène, un alkyle contenant de 1 à 6 atomes de carbone ou un cycloalkyle contenant de 3 à 6 atomes de carbone ;

- A représente un groupe choisi parmi : $-CO-CH_2-$, $-CH(Cl)-CH_2-$, $-CH(OH)-CH_2-$, $-CH_2-CH_2-$, $-CH = CH-$, $-C \equiv C-$ ;

ainsi que leurs sels d'addition avec des acides minéraux ou organiques.

Selon l'invention, par atome d'halogène on entend les atomes de fluor, de chlore, de brome ou d'iode ; l'atome de chlore étant le préféré.

Parmi les cycloalkyles, le cyclohexyle est un groupement préféré.

Ainsi les composés (I) dans lesquels $R_1$ représente un atome de chlore et $R_2$ et $R_3$ représentent chacun un cycloalkyle sont particulièrement préférés.

Lorsque A représente un groupement vinylène, les composés (I) de configuration cis et trans font partie intégrante de l'invention.

Lorsque A représente un groupement chloroéthylène ou hydroxyéthylène , les composés (I) présentent un atome de carbone asymétrique. Les racémates ainsi que les isomères optiquement actifs de ces composés font partie intégrante de l'invention.

Les sels des composés de formule (I) selon la présente invention comprennent aussi bien ceux avec des acides minéraux ou organiques qui permettent une séparation ou une cristallisation convenable des composés de formule (I), tels que l'acide picrique, l'acide oxalique ou un acide optiquement actif, par exemple un acide mandélique ou un acide camphosulfonique, que ceux qui forment des sels pharmaceutiquement acceptables tels que le chlorhydrate, le bromhydrate, le succinate, le sulfate, l'hydrogénosulfate, le dihydrogénophosphate, le méthanesulfonate, le néthylsulfate, l'acétate, le benzoate, le citrate, le glutanate, le maléate, le fumarate, le p-toluènesulfonate, le 2-naphtalènesulfonate.

La présente invention a également pour objet un procédé de préparation des composés (I).

Ce procédé est caractérisé en ce que :

a) on effectue une réaction de condensation avec le formaldéhyde et une amine de formule $HNR_3R_4$ dans laquelle $R_3$ et $R_4$ ont les significations indiquées ci-dessus pour (I) soit sur l'acétophénone de formule :

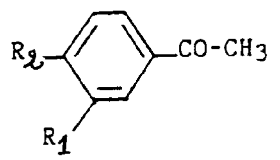

(II)

dans laquelle $R_1$ et $R_2$ ont les significations indiquées ci-dessus pour (I) pour obtenir un composé (I) selon l'invention dans lequel A représente le groupement ($-CO-CH_2-$), soit sur un dérivé phénylacétylénique de formule :

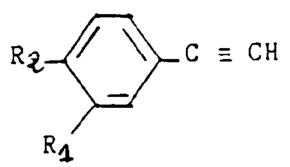

(III)

dans laquelle $R_1$ et $R_2$ sont les significations indiquées ci-dessus, pour obtenir un composé (I) selon l'invention dans lequel A représente le groupement $-C \equiv C-$ ;

b) éventuellement, on fait agir un agent réducteur sur le composé (I) dans lequel A représente un groupement $-CO-CH_2-$ pour préparer le composé (I) selon l'invention dans lequel A représente un groupement $-CHOH-CH_2-$ ;

c) éventuellement, on fait agir sur le composé (I) dans lequel A représente $-CHOH-CH_2-$ un agent chlorant dans un solvant inerte, afin de préparer le composé (I) selon l'invention dans lequel A représente $-CHCl-CH_2-$ ;

d) éventuellement, on effectue une hydrogénation par l'hydrogène naissant du composé (I) dans lequel A représente le groupement acétylénique $-C \equiv C-$ pour préparer le composé (I) dans lequel A représente le groupe $-CH = CH-$ sous forme d'un mélange des isomères cis et trans, ou on effectue une hydrogénation en présence d'un catalyseur métallique sur support pour préparer le composé éthylénique (I) sous forme cis, ou encore on déshydrate le composé (I) dans lequel A représente un groupement $-CHOH-CH_2-$ pour préparer le composé éthylénique (I) sous forme trans ;

e) éventuellement, on effectue une hydrogénation du composé (I) dans lequel A représente un groupement $-CH = CH-$ ou un groupement $-C \equiv C-$, pour préparer le composé (I) selon l'invention dans lequel A repré-

sente le groupement $-CH_2-CH_2-$ ;

f) enfin, si nécessaire, on prépare un sel d'addition d'un composé (I) par addition d'un acide minéral ou organique approprié.

Les acétophénones de départ (II) sont connues ou sont préparées par des méthodes connues telles que celles décrites dans Gazz Chim. Ital. 1949, Tome 79, 453-457 et J. Am. Chem. Soc. 1947, Tome 69, 1651-1652. De même, les amines $HNR_3R_4$ sont connues et disponibles sur le marché.

Lorsque la condensation de l'étape a) du procédé selon l'invention est effectuée sur l'acétophénone (II), on procède en milieu acide dans un solvant comme l'alcool ou le diméthoxyéthane.

On peut notamment obtenir le dérivé phénylacétylénique (III) à partir de l'acétophénone (II) en préparant d'abord un dérivé chloro-phényléthylénique de formule :

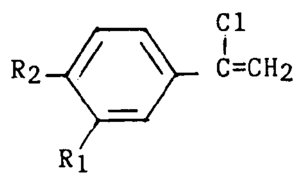

(IV)

par action du pentachlorure de phosphore sur l'acétophénone (II) et hydrolyse puis en effectuant une déshydrohalogénation du composé (IV) en milieu basique.

A partir de l'acétophénone (II), on peut également préparer une semicarbazone intermédiaire (V), puis appliquer le mode opératoire décrit par I. LALEZARI et al. (Angew. Chem., Internat. Ed., 1970, *9* (6) p 464) en faisant agir l'oxyde de sélénium, à chaud, en milieu acide, puis en décomposant à chaud le sélénodiazole intermédiaire (VI) formé et obtenir ainsi le dérivé phénylacétylénique (III) selon le schéma réactionnel suivant :

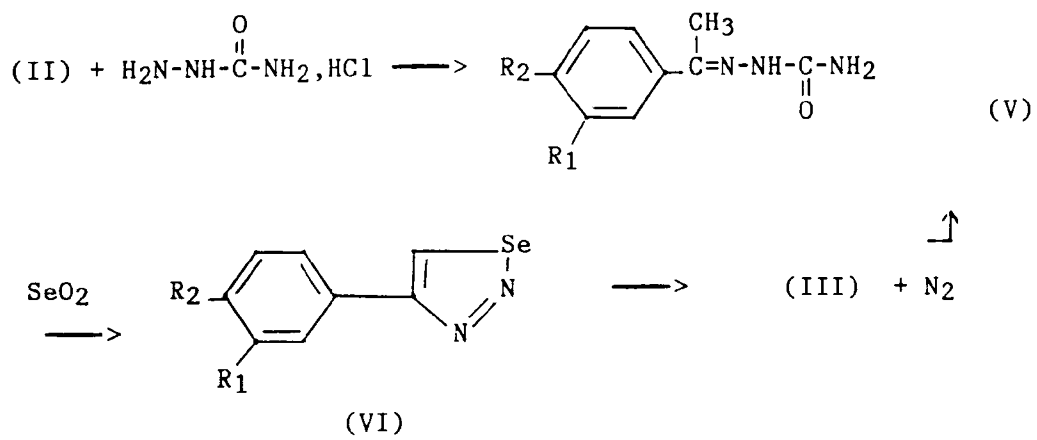


(II) (V) (VI) (III) + N2

Lorsque l'étape a) du procédé selon l'invention est effectuée sur le dérivé phénylacétylénique (III), on procède à chaud, dans un solvant inerte tel que le dioxanne ou le diméthoxyéthane ; pour faciliter la réaction de condensation on peut utiliser comme catalyseur un sel métallique tel que le chlorure cuivreux ou le chlorure cuivrique.

A l'étape b) du procédé, l'agent réducteur est de préférence un hydrure métallique tel que le borohydrure de sodium par exemple, et la réaction est préférentiellement réalisée dans un solvant alcoolique à une température inférieure à 10°C.

A l'étape c) on peut utiliser un agent chlorant comme le chlorure de thionyle, le phosgène ou par exemple un chlorure de phosphore comme l'oxychlorure de phosphore ou le pentachlorure de phosphore.

La réaction est effectuée à chaud dans un solvant comme par exemple le chloroforme ou le dichloroéthane.

A l'étape d) du procédé, l'hydrogénation par hydrogène naissant peut être effectuée par action du zinc dans l'acide acétique, ou bien l'agent de déshydratation est, par exemple, l'acide p-toluène sulfonique utilisé dans le toluène, à température de reflux du milieu ou bien lorsque l'on effectue l'hydrogénation en présence d un catalyseur métallique sur support tel que le palladium sur sulfate de baryum ou sur carbonate de calcium, ou le nickel de Raney, dans un solvant alcoolique ou contenant une partie d'alcool, on peut opérer en présence

de quinoléine pour faciliter la réaction, l'hydrogénation catalytique ainsi réalisée conduit uniquement à des composés (I) de configuration cis (Catalytic Hydrogénation - R.L. Augustine - New-York : Marcel Dekker, 1965, p 69-71).

A l'étape e) du procédé, on peut opérer en présence d'un catalyseur, par exemple l'oxyde de platine.

Le produit de formule (I) est isolé, sous forme de base libre ou de sel, selon les techniques conventionnelles.

Lorsque le composé de formule (I) et obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2-sulfonate.

A la fin de la réaction, le composé de formule (I) peut être isolé sous forme d'un de ses sels, par exemple le chlorhydrate ou l'oxalate ; dans ce cas, s'il est nécessaire, la base libre peut être préparée par neutralisation du dit sel avec une base minérale ou organique telle que l'hydroxyde de sodium ou la triéthylamine ou avec un carbonate ou bicarbonate alcalin, tel que le carbonate ou bicarbonate de sodium ou de potassium.

Les composés de la présente invention ont été essayés dans des tests d'activité immunosuppressive. Notamment, ont été étudiés in vivo sur un modèle expérimental de thyroïdite auto-immune de la souris, induite par la thyroglobuline porcine (TgP) selon J. Salamero et al., Eur. J. Immunol., 1987, *17*, 843-848. Le taux des anticorps anti-thyroglobuline porcine a été mesuré par la méthode ELISA (enzyme-linked immunosorbent assay) après 20 jours consécutifs de traitement. On a observé que les composés selon l'invention induisent chez les animaux traités une nette diminution de la production d'anticorps.

Les composés de formule (I) sont peu toxiques, notamment leur toxicité aigüe est compatible avec leur utilisation comme médicaments dans les domaines thérapeutiques où il est souhaitable de réduire l'activité immunologique. On peut citer, à titre indicatif et non restrictif : les maladies à composante auto-immune telles que, par exemple, la polyarthrite rhumatoïde, le lupus érythémateux, la sclérose en plaque, le diabète ou bien les réactions de rejet de greffes, la réaction du greffon contre l'hôte, les situations de transplantation d'organes (foie, rein, coeur, pancréas, moelle osseuse), le psoriasis.

Pour une telle utilisation, on administre aux mammifères qui nécessitent ledit traitement, une quantité efficace d'un composé de formule (I) ou d'un de ses sels pharmaceutiquement acceptables.

La cis N-cyclohexyl N-éthyl (chloro-3 cyclohexyl-4 phényl)-3 propène-2 ylamine et ses sels pharmaceutiques acceptables, notamment le chlorhydrate, sont particulièrement préférés.

Les composés de formule (I) ci-dessus et leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Les composés de formule (I) sont généralement administrés en unité de dosage. Lesdites unités de dosage sont de préférence formulées sous la forme de compositions pharmaceutiques dans lesquelles le principe actif est mélangé avec un excipient pharmaceutique.

Ainsi selon un autre de ses aspects, la présente invention concerne des compositions pharmaceutiques contenant comme principe actif au moins un des composés de formule (I) ou un de ses sels pharmaceutiquement acceptables.

Dans les compositions pharmaceutiques de la présente invention pour l'administration orale, sublinguale, sous-cutanée, intramusculaire, intraveineuse, transdermique ou rectale, les principes actifs peuvent être administrés sous forme d'unités d'administration, en mélange avec les supports pharmaceutiques classiques, aux animaux et êtres humains. Les formes unitaires d'administration appropriées comprennent les formes par voie orale, telles que les comprimés, les gélules, les poudres, les granules et les solutions ou suspensions orales, les formes d'administration sublinguale et buccale, les formes d'administration transdermique, sous-cutanée, intramusculaire ou intraveineuse et les formes d'administration rectale.

Chaque dose unitaire peut contenir de 0,5 à 1000 mg, de préférence de 2,5 à 200 mg, d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granules dispersibles dans l'eau peuvent contenir l'ingrédient actif en mélange avec des agents de dispersion, des agents mouillants ou des agents de mise en suspension comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Le principe actif peut être formulé également sous forme de microcapsules, éventuellement avec un ou plusieurs supports ou additifs.

La présente invention concerne également les compositions pharmaceutiques contenant au moins un des composés de formule (I) ou un de ses sels pharmaceutiquement acceptables en association avec un autre principe actif. Comme autre principe actif, on pourrait choisir par exemple un immunosuppresseur, un cytostatique ou un antimétabolite tel que notamment, la ciclosporine, l'azathioprine, le méthotrexate, le cyclophosphamide ou le chlorambucil. On pourrait également administrer, en association avec un composé de formule (I) ou un de ses sels pharmaceutiquement acceptables, un anticorps monoclonal anti-rejet, par exemple un anticorps anti-CD3.

Les exemples suivants illustrent l'invention sans toutefois la limiter.

EXEMPLE 1

Chlorhydrate de N-cyclohexyl N-méthyl (chloro-3 cyclohéxyl-4 phényl)-3 propyn-2 ylamine. CM 31739

A) Chloro-3 cyclohéxyl-4 éthynyl-1 benzène.

On ajoute par petites fractions, en 30 minutes, 129 g de pentachlorure de phosphore à 118,3 g de chloro-3 cyclohéxyl-4 acétophénone. En une heure, on monte progressivement la température jusqu'à 105°C, puis on chauffe à cette température pendant 1 heure et demie et à 115°C pendant à nouveau 1 heure et demie. La gomme formée est extraite à l'éther éthylique, la phase éthérée est lavée à la soude à 5 %, séchée et concentrée. On obtient 107 g de chloro-3 cyclohéxyl-4 $\alpha$-chloro styrène. Ce produit est mis en solution dans 450 ml d'éthanol puis chauffé à reflux pendant 24 heures en présence de 94 g de potasse. On concentre la majeure partie de l'alcool que l'on remplace par de l'eau, on extrait à l'éther éthylique, sèche et concentre pour obtenir 72,5 g de produit brut. Après distillation sous pression réduite, on obtient 41,7 g d'un liquide.

Point d'ébullition : 102-104°C sous 4 mm de mercure (= 533 Pa) ;

B) CM 31739

On chauffe à 60°C une solution contenant 16,4 g de chloro-3 cyclohéxyl-4 éthynyl-1 benzène, préparé précédemment dans 30 ml de dioxanne, 4,5 g de paraformaldéhyde et 0,15 g de chlorure cuivreux puis on ajoute en une demi-heure 9,3 g de N-méthyl N-cyclohéxylamine. La température est maintenue à 60°C pendant 1 heure. La réaction terminée, le mélange refroidi est dilué à l'éther et traité par de l'eau puis par une solution d'acide chlorhydrique dilué. La solution acide est ensuite alcalinisée par une solution de soude diluée et on extrait à l'éther. La phase organique est séchée sur sulfate de sodium puis concentrée. On prépare le chlorhydrate à partir de la base brute, on le lave à l'eau puis on cristallise 2 fois dans l'acétonitrile. On obtient 8,4 g du composé attendu.

Fc = 175°C.

EXEMPLE 2

Chlorhydrate de cis N-cyclohexyl N-méthyl (chloro-3 cyclohéxyl-4 phényl)-3 propène-2 ylamine. CM 31748

A température et pression normales, on effectue l'hydrogénation d'une solution contenant 13,1 g du composé préparé à l'exemple 1 sous forme de base, 1,2 g de palladium sur sulfate de baryum à 5 %, dans 100 ml d'acétate d'éthyle et 5 ml de méthanol. Le volume d'hydrogène absorbé est de 650 ml. Après filtration et concentration de la solution, on reprend le résidu dans l'éther éthylique et on précipite le chrorhydrate par barbotage d'acide chlorhydrique gazeux. Après recristallisation dans l'acétonitrile on obtient 5,6 g du composé

attendu.
Rendement 43 %.
Le spectre de RMN de ce composé a été enregistré à 60 MHz dans le diméthylsulfoxyde.

| Déplacement chimique (ppm) | Aspect | Intégration | Attribution |
|---|---|---|---|
| entre 0,75 et 3,5 | massif | 25 H | 2 cyclohexyl $NCH_3$ |
| 3,85 | multiplet | 2 H | $CH_2$ - N |
| 6,2 Jcis = 11 Hz $JCH\text{-}CH_2$ = 6 Hz | doublet de triplet | 1 H | $-CH = CH\text{-}CH_2\text{-}N$ |
| entre 6,5 et 7,5 | massif | 4 H | 3H aromatiques $-CH = CH\text{-}CH_2\text{-}$ |

EXEMPLE 3

Chlorhydrate de cis et trans N-cyclohexyl N-méthyl (chloro-3 cyclohexyl-4 phényl)-3 propène-2 ylamine.

On porte à reflux pendant une heure une solution contenant 6 g de CM 31739, préparé à l'exemple 1, et 6 g de zinc dans 100 ml d'acide acétique et 70 ml d'eau. Après refroidissement, on ajoute de la soude concentrée puis on extrait à l'éther éthylique. La phase éthérée est lavée à l'eau, séchée sur sulfate de sodium puis concentrée. On obtient 5,2 g. On prépare le chlorhydrate par addition d'acide chlorhydrique dans l'éther éthylique et on lave à l'acétone. On recueille 2,8 g de l'isomère cis qui précipite. Le filtrat est concentré, puis après cristallisation dans l'acétone et recristallisation dans l'acétonitrile, on obtient 1,3 g du composé trans (CM 31751).

Point de fusion : 198°C.

Le spectre de RMN du composé trans est enregistré à 60 MHz dans le diméthylsulfoxyde.

| Déplacement chimique | Aspect | Intégration | Attribution |
|---|---|---|---|
| entre 0,9 et 3,4 | massif | 25 H | 2 cyclohexyl $N-CH_3$ |
| 3,75 | multiplet | 2 H | $-CH_2-N$ |
| 6,55 | multiplet | 2 H trans | 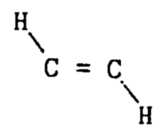 |
| entre 7 et 7,5 | massif | 3 H | H aromatiques |

EXEMPLE 4

Chlorhydrate de N-cyclohexyl N-éthyl (chloro-3 cyclohexyl-4 phényl)-3 propyn-2 ylamine. CM 31738.

A) (chloro-3 cyclohéxyl-4) acétophénone semicarbazone.On dissout 73,59 g de chlorhydrate de semicarbazide et 54,12 g d'acétate de sodium dans 600 ml d'eau distillée. Après agitation du mélange, on ajoute rapidement à température ambiante, 142 g de chloro-3 cyclohéxyl-4 acétophénone dissoute dans 600 ml d'éthanol. On chauffe à 50°C pendant 2 heures, puis on laisse sous agitation à température ambiante pendant une nuit. On essore les cristaux formés, lave à l'eau, à l'acétone puis à l'éther éthylique et sèche sous vide. On obtient 169,50 g de cristaux blancs de semicarbazone.

Rendement : 96 %.

Rf (dichlorométhane-méthanol : 95-5) : 0,4.

La structure de la semicarbazone est confirmée par l'analyse du spectre de RMN.

B) Chloro-3 cyclohéxyl-4 éthynyl-1 benzène.

Dans 400 ml d'acide acétique glacé, on prépare une suspension de 26 g d'oxyde de sélénium finement broyé et 58,7 g de la semicarbazone obtenue à l'étape précédente. On chauffe par un bain d'huile à 60°C pendant 1 heure puis à 80°C pendant 2 heures. Le sélénodiazole intermédiaire est ainsi formé. On augmente la température du bain d'huile à 150°C pendant 3 heures et demie jusqu'à décomposition complète du sélénodiazole et fin du dégagement d'azote. L'acide acétique est évaporé sous vide, le résidu est repris par 600 ml d'éther, filtré pour éliminer le sélénium précipité, lavé 4 fois à l'eau, 1 fois par une solution aqueuse de soude à 5 % et 2 fois à l'eau. On sèche sur sulfate de sodium et carbonate de potassium, évapore sous vide, puis le résidu huileux est distillé sous 0,01 mm de mercure (1,33 Pa) pour donner 24,8 g d'une huile incolore.

Rendement : 57 %.

C) CM 31738

On agite à température ambiante une solution contenant 13,46 g du composé préparé à l'étape précédente et 0,25 g de chlorure cuivrique dans 50 ml de diméthoxyéthane. On ajoute goutte à goutte un

mélange contenant 9 g de formaldéhyde à 35 % dans l'eau et 9,42 g de N-éthyl N-cyclohéxylamine dans 35 ml de diméthoxyéthane. On chauffe à 70 °C pendant 1 heure 15 minutes puis on évapore le solvant sous vide. Le résidu est repris à l'éther, lavé par une solution aqueuse de soude à 5 %, lavé à l'eau puis séché sur sulfate de sodium et concentré à sec. On forme le chlorhydrate par addition d'acide chlorhydrique dans l'éther anhydre, on essore, lave à l'éther éthylique, sèche. Le solide formé est dissous dans le dichlorométhane, lavé 2 fois à l'eau pour éliminer la N-éthyl N-cyclohéxylamine qui n'a pas réagi puis séché et concentré sous vide. Le résidu obtenu est cristallisé dans un mélange acétone-éther. On obtient 22,7 g de cristaux blancs.
Rendement : 93 %
Point de fusion : 169°C
La structure du composé est confirmée par l'analyse du spectre de RMN.

EXEMPLE 5

Chlorhydrate de cis N-cyclohexyl N-éthyl (chloro-3 cyclohexyl-4 phényl)-3 propène-2 ylamine. CM 31747.

18,1 g du composé obtenu à l'exemple 4, sous forme de base libre, sont mis en solution dans 140 ml d'acétate d'éthyle et 5 ml de méthanol. On effectue une hydrogénation, sous pression atmosphérique, en présence de 0,9 g de palladium à 5 % sur sulfate de baryum. L'hydrogénation est arrêtée après 1 heure 40 minutes, le volume d'hydrogène absorbé est de 1,24 litre. On filtre le catalyseur, évapore le solvant et l'on obtient 17 g de produit brut. On chromatographie sur 250 g de silice en éluant par le mélange dichlorométhane-méthanol : 97/3. On obtient 14 g de base libre qui cristallise sous forme de chlorhydrate dans l'éther éthylique. Après filtration et séchage, on obtient 12,22 g du composé attendu, sous forme de chlorhydrate.
Rendement : 61 %
Point de fusion : 192°C
La structure du composé est confirmée par l'analyse du spectre de RMN.

EXEMPLE 6

Chlorhydrate de N,N-dicyclohexyl (chloro-3 cyclohexyl-4 phényl)-3 propyn-2 ylamine. CM 31740.

Ce composé est préparé en suivant le mode opératoire décrit à l'exemple 1 par action de la dicyclohexylamine et du paraformaldéhyde sur le chloro-3 cyclohexyl-4 éthylnyl-1 benzène.
Point de fusion : 165°C.

EXEMPLE 7

Chlorhydrate de cis N,N-dicyclohexyl (chloro-3 cyclohexyl-4 phényl)-3 propène-2 ylamine. CM 31750.

Ce composé est préparé en suivant le mode opératoire décrit à l'exemple 2 à partir du CM 31740, préparé à l'exemple 6, sous forme de base.
Point de fusion : 166°C.

EXEMPLE 8

Chlorhydrate de N-cyclohexyl N-éthyl (chloro-3 cyclohexyl-4 phényl)-3 propylamine. SR 45596 A.

Ce composé est obtenu à partir du CM 31738 préparé à l'exemple 4.

On dissout 4 g du chlorhydrate préparé à l'exemple 4 dans 1 ml de méthanol et 28 ml d'acétate d'éthyle et l'on ajoute 0,18 g de sulfate de baryum sur palladium.

Le milieu réactionnel est placé sous atmosphère d'hydrogène pendant 7 heures puis on filtre le catalyseur et on concentre le milieu à sec sous vide. Le résidu est repris par du dichlorométhane et chromatographié sur une colonne de silice en éluant par un mélange dichlorométhane-méthanol (93-7, v/v) puis (90-10, v/v).

Après évaporation, l'huile obtenue est diluée dans l'éther puis le sel cristallise par addition d'éther chlorhydrique. On obtient 1,5 g.
Point de fusion = 165°C.

EXEMPLE 9

Chlorhydrate de (chloro-3 cyclohexyl-4 phényl)-1 (N-cyclohexyl N-éthylamino)-3 propanone-1. SR 46232 A.

On porte à reflux pendant 18 heures 23,6 g de (chloro-3 cyclohexyl-4) acétophénone 16,3 g de chlorhydrate de N-cyclohexyl N-éthylamine, 6 g de paraformaldéhyde et 3,5 ml d'acide chlorhydrique dans 200 ml de diméthoxyéthane. On évapore le solvant et reprend le résidu par 700 ml d'éther éthylique. Le solide obtenu est repris au dichlorométhane, lavé à l'eau, essoré, séché sur sulfate de sodium. L'huile jaune obtenue est reprise par 500 ml d'acétate d'éthyle. On obtient 21,5 g d'un solide blanc qui cristallise.

Point de fusion : 154-156°C.

EXEMPLE 10

Chlorhydrate de (chloro-3 cyclohexyl-4 phényl)-1 (N-cyclohexyl N-éthyl amino)-3 propanol-1. SR 46233 A.

On refroidit à 4°C, 4,12 g de la propanone préparée à l'exemple précédent dans 100 ml de méthanol. On ajoute par portions 0,13 g de borohydrure de sodium, en maintenant la température entre 5° et 10°C pendant 30 minutes puis on laisse revenir à température ambiante pendant 1 heure. On évapore le méthanol, reprend par de l'eau puis extrait à l'acétate d'éthyle et sèche sur sulfate de sodium. On prépare le chlorhydrate par addition d'acide chlorhydrique gazeux. Le solide blanc qui cristallise est lavé à l'éther. On obtient 3,23 g du produit attendu.

Point de fusion : 165-167°C.

EXEMPLE 11

Chlorhydrate de chloro-3 (chloro-3 cyclohexyl-4 phényl)-3 N-cyclohexyl N-éthyl propylamine. SR 46264 A.

On porte à reflux un mélange contenant 6,2 g du composé préparé à l'exemple précédent et 6,3 g de chlorure de thionyle dans 150 ml de chloroforme. Après 20 minutes le dégagement gazeux cesse, on évapore le milieu réactionnel, lave le résidu huileux à l'acétone puis ajoute 150 ml d'acétate d'éthyle. Le produit attendu cristallise. On obtient 5,8 g.

Point de fusion : 174-176°C.

EXEMPLES 12 et 13

En suivant respectivement les modes opératoires décrits dans les exemples 9 et 10, on prépare les composés suivants :

- Oxylate de (chloro-3 cyclohexyl-4 phényl)-1 N-cyclohexylamino-3 propanone-1.
  Point de fusion : 181-183°C
- Chlorhydrate de (chloro-3 cyclohexyl-4 phényl)-1 N-cyclohexylamino-3 propanol-1.
  Point de fusion : 264-266°C.

EXEMPLE 14

Préparation de gélules :

| | |
|---|---|
| CM 31747 | 25 mg |
| Lactose | 110 mg |
| Stéréate de magnésium | 5 mg |

## Revendications

### Revendications pour les Etats contractants suivants : AT, BE, CH, DE, FR, GB, IT, LI, LU, NL, SE

**1.** Composé de formule :

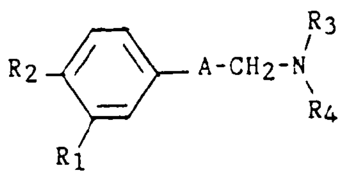

(I)

dans laquelle :

- $R_1$ représente un atome d'hydrogène ou un atome d'halogène ;
- $R_2$ représente un cyclohéxyle ou un phényle ;
- $R_3$ représente un cycloalkyle contenant de 3 à 6 atomes de carbone ;
- $R_4$ représente un atome d'hydrogène, un alkyle contenant de 1 à 6 atomes de carbone ou un cycloalkyle contenant de 3 à 6 atomes de carbone ;
- A représente un groupe choisi parmi : $-CO-CH_2-$, $-CH(Cl)-CH_2-$, $-CH(OH)-CH_2-$, $-CH_2-CH_2-$, $-CH=CH-$, $-C \equiv C-$ ;

ou un de ses sels d'addition avec des acides minéraux ou organiques.

**2.** Sels d'addition pharmaceutiquement acceptables d'un composé selon la revendication 1.

**3.** La cis N-cyclohexyl N-éthyl (chloro-3 cyclohexyl-4 phényl)-3 propène-2-ylamine ou un de ses sels d'addition pharmaceutiquement acceptables.

**4.** Le chlorhydrate de cis N-cyclohexyl N-éthyl (chloro-3 cyclohexyl-4 phényl)-3 propène-2 ylamine.

**5.** Procédé de préparation des composés (I) selon l'une quelconque des revendications 1 à 4 caractérisé en ce que :

a) on effectue une réaction de condensation avec le formaldéhyde et une amine de formule $HNR_3R_4$ dans laquelle $R_3$ et $R_4$ ont les significations indiquées à la revendication 1 pour (I) soit sur l'acétophénone de formule :

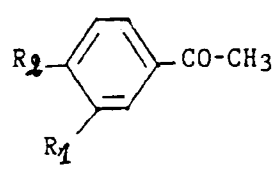

(II)

dans laquelle $R_1$ et $R_2$ ont les significations indiquées à la revendication (I) pour obtenir un composé (I), dans lequel A représente le groupement ($-CO-CH_2-$), soit sur un dérivé phénylacétylénique de formule :

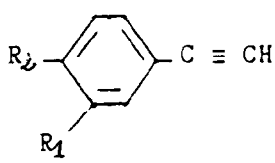

(III)

dans laquelle $R_1$ et $R_2$ ont les significations indiqués à la revendication pour obtenir un composé (I) dans lequel A représente le groupement $-C \equiv C-$ ;

b) éventuellement, on fait agir un agent réducteur sur le composé (I) dans lequel A représente un groupement $-CO-CH_2-$ pour préparer le composé (I)dans lequel A représente un groupement $-CHOH-CH_2-$ ;

c) éventuellement, on fait agir sur le composé (I) dans lequel A représente $-CHOH-CH_2-$ un agent chlorant dans un solvant inerte, afin de préparer le composé (I)dans lequel A représente $-CHCl-CH_2-$ ;

d) éventuellement, on effectue une hydrogénation par l'hydrogène naissant du composé (I) dans lequel A représente le groupement acétylénique $-C \equiv C-$ pour préparer le composé (I) dans lequel A représente le groupe $-CH=CH-$ sous forme d'un mélange des isomères cis et trans, ou on effectue une hydrogénation en présence d'un catalyseur métallique sur support pour préparer le composé éthylénique (I)

sous forme cis, ou encore on déshydrate le composé (I) dans lequel A représente un groupement -CHOH-$CH_2$- pour préparer le composé éthylénique (I) sous forme trans ;

e) éventuellement, on effectue une hydrogénation du composé (I) dans lequel A représente un groupement -CH = CH- ou un groupement -C ≡ C-, pour préparer le composé (I) selon l'invention dans lequel A représente le groupement -$CH_2$-$CH_2$- ;

f) enfin, si nécessaire, on prépare un sel d'addition d'un composé (I) par addition d'un acide minéral ou organique approprié.

**6.** Composition pharmaceutique contenant, en tant que principe actif, au moins un composé selon l'une quelconque des revendications 1, 2, 3 ou 4.

**7.** Composition pharmaceutique selon la revendication 6 caractérisée en ce qu'elle est sous forme d'unité de dosage, dans laquelle le principe actif est mélangé à un excipient pharmaceutique.

**8.** Composition pharmaceutique selon l'une des revendications 6 ou 7 caractérisée en ce qu'elle contient de 0,5 à 1000 mg de principe actif par unité de dosage.

**9.** Composition pharmaceutique selon l'une des revendications 7 ou 8 caractérisée en ce qu'elle contient 2,5 à 200 mg de principe actif par unité de dosage.

**Revendications pour l'Etat contractant suivant : ES**

**1.** Procédé de préparation d'un composé de formule :

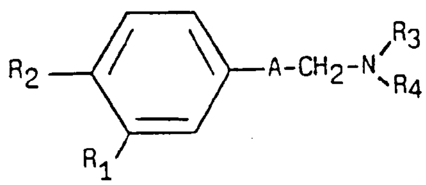

(I)

dans laquelle :

- $R_1$ représente un atome d'hydrogène ou un atome d'halogène ;
- $R_2$ représente un cyclohéxyle ou un phényle ;
- $R_3$ représente un cycloalkyle contenant de 3 à 6 atomes de carbone ;
- $R_4$ représente un atome d'hydrogène, un alkyle contentant de 1 à 6 atomes de carbone ou un cycloalkyle contenant de 3 à 6 atomes de carbone ;
- A représente un groupe choisi parmi : -CO-$CH_2$, -CH(Cl)-$CH_2$-, -CH(OH)-$CH_2$-, -$CH_2$-$CH_2$-, -CH = CH-, -C≡C- ;

ou d'un de ses sels d'addition avec des acides minéraux ou organiques, caractérisé en ce que :

a) on effectue une réaction de condensation avec le formaldéhyde et une amine de formule $HNR_3R_4$ dans laquelle $R_3$ et $R_4$ ont les significations indiquées ci-dessus pour (I) soit sur l'acétophénone de formule :

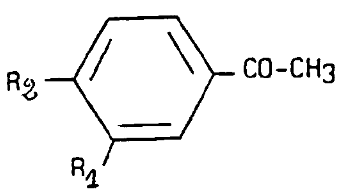

(II)

dans laquelle $R_1$ et $R_2$ ont les significations indiquées ci-dessus pour (I) pour obtenir un composé (I) dans lequel A représente le groupement (-CO-$CH_2$-), soit sur un dérivé phénylacétylénique de formule :

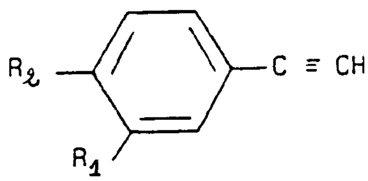

(III)

dans laquelle $R_1$ et $R_2$ ont les significations indiquées ci-dessus pour (I),

pour obtenir un composé (I) dans lequel A représente le groupement -C≡C- ;

b) éventuellement, on fait agir un agent réducteur sur le composé (I) dans lequel A représente un groupement $-CO-CH_2-$ pour préparer le composé (I) dans lequel A représente un groupement $-CHOH-CH_2-$ ;

c) éventuellement, on fait agir sur le composé (I) dans lequel A représente $-CHOH-CH_2-$ un agent chlorant dans un solvant inerte, afin de préparer le composé (I) dans lequel A représente $-CHCl-CH_2-$ ;

d) éventuellement, on effectue une hydrogénation par l'hydrogène naissant du composé (I) dans lequel A représente le groupement acétylénique -C = C- pour préparer le composé (I) dans lequel A représente le groupe -CH ≡ CH- sous forme d'un mélange des isomères cis et trans, ou on effectue une hydrogénation en présence d'un catalyseur métallique sur support pour préparer le composé éthylénique (I) sous forme cis, ou encore on déshydrate le composé (I) dans lequel A représente un groupement $-CHOH-CH_2-$ pour préparer le composé éthylénique (I) sous forme trans ;

e) éventuellement, on effectue un hydrogénation du composé (I) dans lequel A représente un groupement -CH = CH- ou un groupement -C ≡ C-, pour préparer le composé (I) selon l'invention dans lequel A représente le groupement $-CH_2-CH_2-$ ;

f) enfin, si nécessaire, on prépare un sel d'addition d'un composé (I) par addition d'un acide minéral ou organique approprié.

**2.** Procédé selon la revendication 1, caractérisé en ce qu'à l'étape f) on prépare un sel d'addition pharmaceutiquement acceptable.

**3.** Procédé selon l'une des revendications 1 ou 2, caractérisé en ce que :

- l'on effectue une réaction de condensation avec le formaldéhyde et une amine de formule :

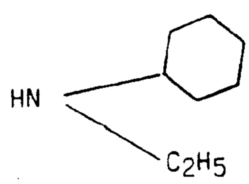

sur le dérivé phénylacétylénique de formule :

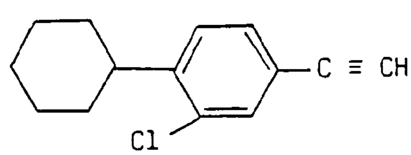

pour obtenir le composé de formule :

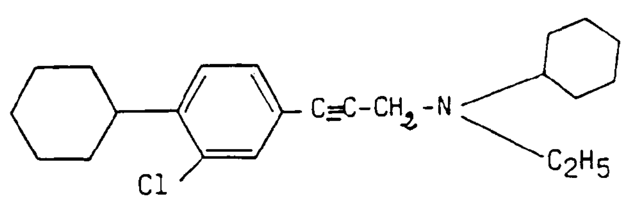

et

- en ce que l'on hydrogène ledit composé obtenu en présence d'un catalyseur métallique sur support pour préparer le composé de formule :

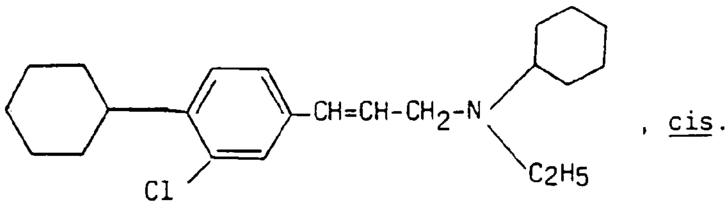

isolé sous forme de base ou de chlorhydrate.

**4.** Procédé de préparation de compositions pharmaceutiques caractérisé en ce qu'il consiste à mélanger à un excipient pharmaceutique au moins un composé de formule (I) préparé selon l'une quelconque des revendications 1 à 3.

**5.** Procédé selon la revendication 4 pour la préparation d'unités de dosage.

**6.** Procédé selon la revendication 5 pour la préparation d'unités de dosage contenant de 0,5 à 1000 mg de principe actif de formule (I).

**7.** Procédé selon la revendication 5 pour la préparation d'unités de dosage contenant de 2,5 à 200 mg de principe actif de formule (I).

**Revendications pour l'Etat contractant suivant : GR**

**1.** Composé de formule :

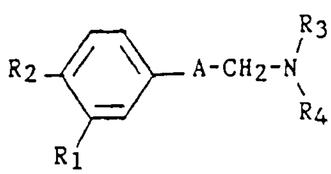

(I)

dans laquelle :

- $R_1$ représente un atome d'hydrogène ou un atome d'halogène ;
- $R_2$ représente un cyclohéxyle ou un phényle ;
- $R_3$ représente un cycloalkyle contenant de 3 à 6 atomes de carbone ;
- $R_4$ représente un atome d'hydrogène, un alkyle contenant de 1 à 6 atomes de carbone ou un cycloalkyle contenant de 3 à 6 atomes de carbone ;
- A représente un groupe choisi parmi : $-CO-CH_2-$, $-CH(Cl)-CH_2-$, $-CH(OH)-CH_2-$, $-CH_2-CH_2-$, $-CH=CH-$, $-C \equiv C-$ ;

ou un de ses sels d'addition avec des acides minéraux ou organiques.

**2.** Sels d'addition pharmaceutiquement acceptables d'un composé selon la revendication 1.

**3.** La cis N-cyclohexyl N-éthyl (chloro-3 cyclohexyl-4 phényl)-3 propène-2-ylamine ou un de ses sels d'addition pharmaceutiquement acceptables.

**4.** Le chlorhydrate de cis N-cyclohexyl N-éthyl (chloro-3 cyclohexyl-4 phényl)-3 propène-2 ylamine.

**5.** Procédé de préparation des composés (I) selon l'une quelconque des revendications 1 à 4 caractérisé en ce que :

a) on effectue une réaction de condensation avec le formaldéhyde et une amine de formule $HNR_3R_4$ dans laquelle $R_3$ et $R_4$ ont les significations indiquées à la revendication 1 pour (I) soit sur l'acétophénone de formule :

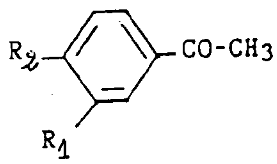

(II)

dans laquelle $R_1$ et $R_2$ ont les significations indiquées à la revendication (I) pour obtenir un composé (I), dans lequel A représente le groupement ($-CO-CH_2-$), soit sur un dérivé phénylacétylénique de formule :

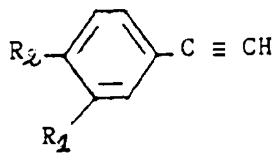

(III)

dans laquelle $R_1$ et $R_2$ ont les significations indiqués à la revendication pour obtenir un composé (I) dans lequel A représente le groupement -C≡C- ;
b) éventuellement, on fait agir un agent réducteur sur le composé (I) dans lequel A représente un groupement $-CO-CH_2-$ pour préparer le composé (I) dans lequel A représente un groupement $-CHOH-CH_2-$ ;
c) éventuellement, on fait agir sur le composé (I) dans lequel A représente $-CHOH-CH_2-$ un agent chlorant dans un solvant inerte, afin de préparer le composé (I)dans lequel A représente $-CHCl-CH_2-$ ;
d) éventuellement, on effectue une hydrogénation par l'hydrogène naissant du composé (I) dans lequel A représente le groupement acétylénique -C ≡ C- pour préparer le composé (I) dans lequel A représente le groupe -CH = CH- sous forme d'un mélange des isomères cis et trans, ou on effectue une hydrogénation en présence d'un catalyseur métallique sur support pour préparer le composé éthylénique (I) sous forme cis, ou encore on déshydrate le composé (I) dans lequel A représente un groupement $-CHOH-CH_2-$ pour préparer le composé éthylénique (I) sous forme trans ;
e) éventuellement, on effectue une hydrogénation du composé (I) dans lequel A représente un groupement -CH = CH- ou un groupement -C = C-, pour préparer le composé (I) selon l'invention dans lequel A représente le groupement $-CH_2-CH_2$ ;
f) enfin, si nécessaire, on prépare un sel d'addition d'un composé (I) par addition d'un acide minéral ou organique approprié.

6. Procédé de préparation de compositions pharmaceutiques caractérisé en ce qu'il consiste à mélanger à un excipient pharmaceutique au moins un composé de formule (I) selon l'une quelconque des revendications 1 à 4.

7. Procédé selon la revendication 6 pour la préparation d'unités de dosage.

8. Procédé selon la revendication 7 pour la préparation d'unités de dosage contenant de 0,5 à 1000 mg de principe actif de formule (I).

9. Procédé selon la revendication 7 pour la préparation d'unités de dosage contenant de 2,5 à 200 mg de principe actif de formule (I).

## Patentansprüche

### Patentansprüche für folgende Vertragsstaaten : AT, BE, CH, DE, FR, GB, IT, LI, LU, NL, SE

1. Verbindung der Formel:

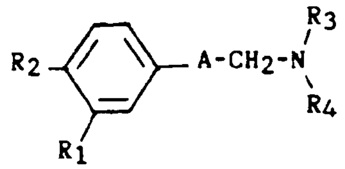

(I)

worin:

- $R_1$ ein Wasserstoffatom oder ein Halogenatom darstellt;
- $R_2$ ein Cyclohexyl oder ein Phenyl darstellt;
- $R_3$ ein Cycloalkyl mit 3 bis 6 Kohlenstoffatomen darstellt;
- $R_4$ ein Wasserstoffatom, ein Alkyl mit 1 bis 6 Kohlenstoffatomen oder ein Cycloalkyl mit 3 bis 6 Kohlenstoffatomen darstellt;
- A eine Gruppe darstellt, ausgewählt aus: $-CO-CH_2-$, $-CH(Cl)-CH_2-$, $-CH(OH)-CH_2-$, $-CH_2-CH_2-$, $-CH=CH-$, $-C\equiv C-$ ;

oder eines ihrer Additionssalze mit Mineral- oder organischen Säuren.

2. Pharmazeutisch akzeptable Additionssalze einer Verbindung nach Anspruch 1.

3. 3-N-Cyclohexyl-N-ethyl-(3-chlor-4-cyclohexylphenyl)-prop-cis-2-enylamin oder eines seiner pharmazeutisch akzeptablen Additionssalze.

4. 3-N-Cyclohexyl-N-ethyl-(3-chlor-4-cyclohexylphenyl)-prop-cis-2-enylamin-hydrochlorid.

5. Verfahren zur Herstellung der Verbindungen (I) nach einem der Ansprüche 1 bis 4, dadurch gekennzeichnet, daß

a) man eine Kondensationsreaktion mit Formaldehyd und einem Amin der Formel $HNR_3R_4$ ausführt, worin $R_3$ und $R_4$ die in Anspruch 1 für (I) angegebenen Bedeutungen haben, entweder mit einem Acetophenon der Formel:

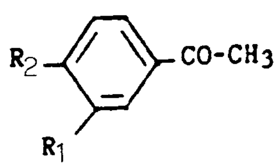

(II)

worin $R_1$ und $R_2$ die in Anspruch 1 für (I) angegebenen Bedeutungen haben, um eine Verbindung (I) zu erhalten, in der A die Gruppe ($-CO-CH_2$) darstellt, oder mit einem Phenylacetylenderivat der Formel:

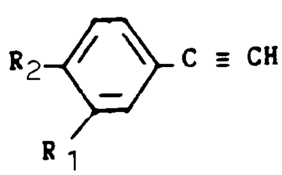

(III)

worin $R_1$ und $R_2$ die in Anspruch 1 angegebenen Bedeutungen haben, um eine Verbindung (I) zu erhalten, in der A die Gruppe $-C\equiv C-$ darstellt ;

b) man gegebenenfalls ein Reaktionsmittel auf die Verbindung (I), in der A eine Gruppe $-CO-CH_2-$ darstellt, wirken läßt, um die Verbindung (I) herzustellen, in der A eine Gruppe $-CHOH-CH_2-$ bedeutet;

c) man gegebenenfalls auf die Verbindung (I), in der A für $-CHOH-CH_2-$ steht, ein Chlorierungsmittel in einem inerten Lösungsmittel wirken läßt, um die Verbindung (I) herzustellen, in der A $-CHCl-CH_2-$ bedeutet;

d) man gegebenenfalls eine Hydrierung mit dem naszierenden Wasserstoff der Verbindung (I), in der A die Acetylengruppe $-C\equiv C-$ darstellt, ausführt, um die Verbindung (I), in der A die Gruppe $-CH=CH-$ darstellt, in Form einer Mischung von cis- und trans-Isomeren zu erhalten, oder man eine Hydrierung in Gegenwart eines metallischen Katalysators auf einem Träger durchführt, um die Ethylenverbindung

(I) in cis-Form zu erhalten, oder aber man die Verbindung (I), in der A eine Gruppe $-CHOH-CH_2-$ darstellt, zur Herstellung der Ethylenverbindung (I) in trans-Form dehydratisiert;
e) man gegebenenfalls eine Hydrierung der Verbindung (I), in der A eine Gruppe -CH=CH- oder eine Gruppe -C≡C- darstellt, durchführt, um die erfindungsgemäße Verbindung (I) herzustellen, in der A die Gruppe $-CH_2-CH_2-$ bedeutet;
f) man schließlich notwendigenfalls durch Zugabe einer geeigneten Mineral- oder organischen Säure ein Additionssalz einer Verbindung (I) herstellt.

6. Pharmazeutische Zusammensetzung, die als Wirkstoff mindestens eine Verbindung nach einem der Ansprüche 1, 2, 3 oder 4 enthält.

7. Pharmazeutische Zusammensetzung nach Anspruch 6, dadurch gekennzeichnet, daß sie in Dosiseinheitsform vorliegt, wobei der Wirkstoff mit einem pharmazeutischen Exzipienten vermischt ist.

8. Pharmazeutische Zusammensetzung nach einem der Ansprüche 6 oder 7, dadurch gekennzeichnet, daß sie 0,5 bis 1000 mg Wirkstoff pro Dosiseinheit enthält.

9. Pharmazeutische Zusammensetzung nach einem der Ansprüche 6 oder 7, dadurch gekennzeichnet, daß sie 2,5 bis 200 mg Wirkstoff pro Dosiseinheit enthält.

**Patentansprüche für folgenden Vertragsstaat : ES**

1. Verfahren zur Herstellung einer Verbindung der Formel:

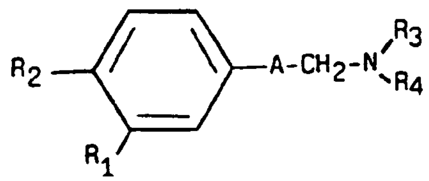

(I)

worin:
- $R_1$ ein Wasserstoffatom oder ein Halogenatom darstellt;
- $R_2$ ein Cyclohexyl oder ein Phenyl darstellt;
- $R_3$ ein Cycloalkyl mit 3 bis 6 Kohlenstoffatomen darstellt;
- $R_4$ ein Wasserstoffatom, ein Alkyl mit 1 bis 6 Kohlenstoffatomen oder ein Cycloalkyl mit 3 bis 6 Kohlenstoffatomen darstellt;
- A eine Gruppe darstellt, ausgewählt aus: $-CO-CH_2-$, $-CH(Cl)-CH_2-$, $-CH(OH)-CH_2-$, $-CH_2-CH_2-$, -CH = CH-, -C ≡ C- ;

oder eines ihrer Additionssalze mit Mineral- oder organischen Säuren, dadurch gekennzeichnet, daß.
a) man eine Kondensationsreaktion mit Formaldehyd und einem Amin der Formel $HNR_3R_4$ ausführt, worin $R_3$ und $R_4$ die in oben für (I) angegebenen Bedeutungen haben, entweder mit einem Acetophenon der Formel:

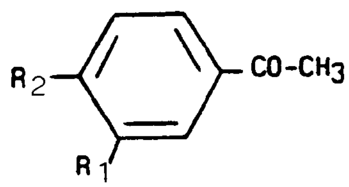

(II)

worin $R_1$ und $R_2$ die oben für (I) angegebenen Bedeutungen haben, um eine Verbindung (I) zu erhalten, in der A die Gruppe $(-CO-CH_2-)$ darstellt, oder mit einem Phenylacetylenderivat der Formel:

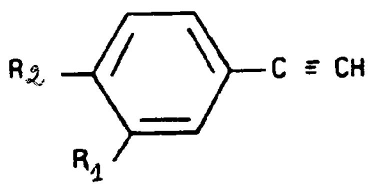

(III)

worin $R_1$ und $R_2$ die oben für (I) angegebenen Bedeutungen haben, um eine Verbindung (I) zu erhalten, in der A die Gruppe -C≡C- darstellt ;

b) man gegebenenfalls ein Reaktionsmittel auf die Verbindung (I), in der A eine Gruppe $-CO-CH_2-$ darstellt, wirken läßt, um die Verbindung (I) herzustellen, in der A eine Gruppe $-CHOH-CH_2-$ bedeutet;

c) man gegebenenfalls auf die Verbindung (I), in der A für $-CHOH-CH_2-$ steht, ein Chlorierungsmittel in einem inerten Lösungsmittel wirken läßt, um die Verbindung (I) herzustellen, in der A $-CHCl-CH_2-$ bedeutet;

d) man gegebenenfalls eine Hydrierung mit dem naszierenden Wasserstoff der Verbindung (I), in der A die Acetylengruppe -C≡C- darstellt, ausführt, um die Verbindung (I), in der A die Gruppe -CH=CH- darstellt, in Form einer Mischung von cis- und trans-Isomeren zu erhalten, oder man eine Hydrierung in Gegenwart eines metallischen Katalysators auf einem Träger durchführt, um die Ethylenverbindung (I) in cis-Form zu erhalten, oder aber man die Verbindung (I), in der A eine Gruppe $-CHOH-CH_2-$ darstellt, zur Herstellung der Ethylenverbindung (I) in trans-Form dehydratisiert;

e) man gegebenenfalls eine Hydrierung der Verbindung (I), in der A eine Gruppe -CH=CH- oder eine Gruppe -C≡C- darstellt, durchführt, um die erfindungsgemäße Verbindung (I) herzustellen, in der A die Gruppe $-CH_2-CH_2-$ bedeutet;

f) man schließlich notwendigenfalls durch Zugabe einer geeigneten Mineral- oder organischen Säure ein Additionssalz einer Verbindung (I) herstellt.

**2.** Verfahren nach Anspruch 1, dadurch gekennzeichnet, daß man in Stufe f) ein pharmazeutisch akzeptables Additionssalz herstellt.

**3.** Verfahren nach einem der Ansprüche 1 oder 2, dadurch gekennzeichnet, daß man:
- eine Kondensationsreaktion mit Formaldehyd und einem Amin der Formel

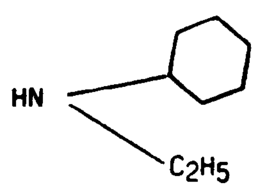

mit einem Phenylacetylenderivat der Formel:

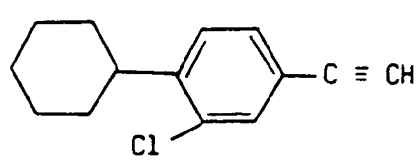

durchführt, um die Verbindung der Formel:

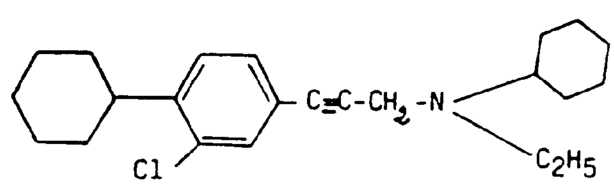

zu erhalten,
- und daß man die erhaltene Verbindung in Gegenwart eines metallischen Katalysators auf einem Träger hydriert, um die Verbindung der Formel:

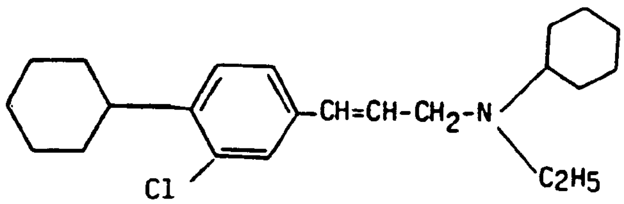

, cis

isoliert in Basen- oder Hydrochloridform, herzustellen.

4. Verfahren zur Herstellung von pharmazeutischen Zusammensetzungen, dadurch gekennzeichnet, daß es darin besteht, mindestens eine Verbindung nach einem der Ansprüche 1 bis 3 mit einem pharmazeutischen Exzipienten zu vermischen.

5. Verfahren nach Anspruch 4 zur Herstellung von Dosiseinheitsformen.

6. Verfahren nach Anspruch 5 zur Herstellung von Dosiseinheiten, die 0,5 bis 1000 mg Wirkstoff der Formel (I) enthalten.

7. Verfahren nach Anspruch 5 zur Herstellung von Dosiseinheiten, die 2,5 bis 200 mg Wirkstoff der Formel (I) enthalten.

**Patentansprüche für folgenden Vertragsstaat : GR**

1. Verbindung der Formel:

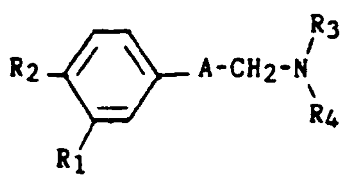

(I)

worin:
- $R_1$ ein Wasserstoffatom oder ein Halogenatom darstellt;
- $R_2$ ein Cyclohexyl oder ein Phenyl darstellt;
- $R_3$ ein Cycloalkyl mit 3 bis 6 Kohlenstoffatomen darstellt;
- $R_4$ ein Wasserstoffatom, ein Alkyl mit 1 bis 6 Kohlenstoffatomen oder ein Cycloalkyl mit 3 bis 6 Kohlenstoffatomen darstellt;
- A eine Gruppe darstellt, ausgewählt aus: $-CO-CH_2-$, $-CH(Cl)-CH_2-$, $-CH(OH)-CH_2-$, $-CH_2-CH_2-$, $-CH=CH-$, $-C\equiv C-$;
oder eines ihrer Additionssalze mit Mineral- oder organischen Säuren.

2. Pharmazeutisch akzeptable Additionssalze einer Verbindung nach Anspruch 1.

3. 3-N-Cyclohexyl-N-ethyl-(3-chlor-4-cyclohexylphenyl)-prop-cis-2-enylamin oder eines seiner pharmazeutisch akzeptablen Additionssalze.

4. 3-N-Cyclohexyl-N-ethyl-(3-chlor-4-cyclohexylphenyl)-prop-cis-2-enylamin-hydrochlorid.

5. Verfahren zur Herstellung der Verbindungen (I) nach einem der Ansprüche 1 bis 4, dadurch gekennzeichnet, daß
a) man eine Kondensationsreaktion mit Formaldehyd und einem Amin der Formel $HNR_3R_4$ ausführt, worin $R_3$ und $R_4$ die in Anspruch 1 für (I) angegebenen Bedeutungen haben, entweder mit einem Acetophenon der Formel:

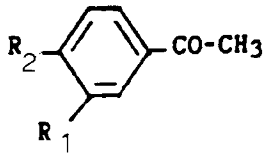

(II)

worin $R_1$ und $R_2$ die in Anspruch 1 für (I) angegebenen Bedeutungen haben, um eine Verbindung (I) zu erhalten, in der A die Gruppe ($-CO-CH_2$) darstellt, oder mit einem Phenylacetylenderivat der Formel:

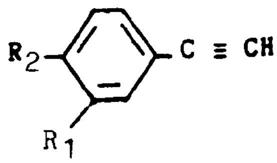

(III)

worin $R_1$ und $R_2$ die in Anspruch 1 angegebenen Bedeutungen haben, um eine Verbindung (I) zu erhalten, in der A die Gruppe -C≡C-darstellt ;
b) man gegebenenfalls ein Reaktionsmittel auf die Verbindung (I), in der A eine Gruppe $-CO-CH_2-$ darstellt, wirken läßt, um die Verbindung (I) herzustellen, in der A eine Gruppe $-CHOH-CH_2$-bedeutet ;
c) man gegebenenfalls auf die Verbindung (I), in der A für $-CHOH-CH_2-$ steht, ein Chlorierungsmittel in einem inerten Lösungsmittel wirken läßt, um die Verbindung (I) herzustellen, in der A $-CHCl-CH_2-$ bedeutet;
d) man gegebenenfalls eine Hydrierung mit dem naszierenden Wasserstoff der Verbindung (I), in der A die Acetylengruppe -C≡C- darstellt, ausführt, um die Verbindung (I), in der A die Gruppe -CH=CH- darstellt, in Form einer Mischung von cis- und trans-Isomeren zu erhalten, oder man eine Hydrierung in Gegenwart eines metallischen Katalysators auf einem Träger durchführt, um die Ethylenverbindung (I) in cis-Form zu erhalten, oder aber man die Verbindung (I), in der A eine Gruppe $-CHOH-CH_2-$ darstellt, zur Herstellung der Ethylenverbindung (I) in trans-Form dehydratisiert;
e) man gegebenenfalls eine Hydrierung der Verbindung (I), in der A eine Gruppe -CH=CH- oder eine Gruppe -C≡C- darstellt, durchführt, um die erfindungsgemäße Verbindung (I) herzustellen, in der A die Gruppe $-CH_2-CH_2-$ bedeutet;
f) man schließlich notwendigenfalls durch Zugabe einer geeigneten Mineral- oder organischen Säure ein Additionssalz einer Verbindung (I) herstellt.

**6.** Verfahren zur Herstellung von pharmazeutischen Zusammensetzungen, dadurch gekennzeichnet, daß es darin besteht, mindestens eine Verbindung nach einem der Ansprüche 1 bis 4 mit einem pharmazeutischen Exzipienten zu vermischen.

**7.** Verfahren nach Anspruch 6 zur Herstellung von Dosiseinheitsformen.

**8.** Verfahren nach Anspruch 7 zur Herstellung von Dosiseinheiten, die 0,5 bis 1000 mg Wirkstoff der Formel (I) enthalten.

**9.** Verfahren nach Anspruch 7 zur Herstellung von Dosiseinheiten, die 2,5 bis 200 mg Wirkstoff der Formel (I) enthalten.

## Claims

**Claims for the following Contracting States : AT, BE, CH, DE, FR, GB, IT, LI, LU, NL, SE**

**1.** A compound of the formula

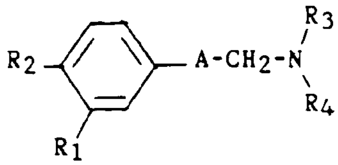

(I)

in which:

- $R_1$ represents a hydrogen atom or a halogen atom;
- $R_2$ represents a cyclohexyl or a phenyl;
- $R_3$ represents a cycloalkyl containing from 3 to 6 carbon atoms;
- $R_4$ represents a hydrogen atom, an alkyl containing from 1 to 6 carbon atoms or a cycloalkyl containing from 3 to 6 carbon atoms; and
- A represents a group selected from : $-CO-CH_2-$, $-CH(Cl)-CH_2-$, $-CH(OH)-CH_2-$, $-CH_2-CH_2-$, $-CH=CH-$ and $-C{\equiv}C-$,

or one of its addition salts with mineral or organic acids.

2. Pharmaceutically acceptable addition salts of a compound according to claim 1.

3. Cis-N-cyclohexyl-N-ethyl-3-(3-chloro-4-cyclohexyl-phenyl)prop-2-enylamine or one of its pharmaceutically acceptable addition salts.

4. Cis-N-cyclohexyl-N-ethyl-3-(3-chloro-4-cyclohexyl-phenyl)prop-2-enylamine hydrochloride.

5. A method of preparing the compounds (I) according to any one of claims 1 to 4, characterized in that it comprises:

a) carrying out a condensation reaction of formaldehyde and an amine of the formula $HNR_3R_4$, in which $R_3$ and $R_4$ are as defined in claim 1 for (I), either with the acetophenone of the formula

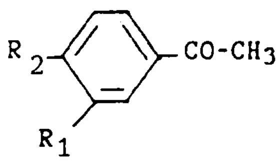

(II)

in which $R_1$ and $R_2$ are as defined in claim 1 for (I), to give a compound (I), in which A represents the group $-CO-CH_2-$, or with a phenylacetylenic derivative of formula

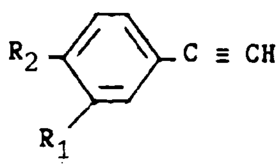

(III)

in which $R_1$ and $R_2$ are as defined in claim 1, to give a compound (I) in which A represents a group $-C{\equiv}C-$;

b) if appropriate, reacting a reducing agent with the compound (I) in which A represents a group $-CO-CH_2-$ in order to prepare the compound (I) in which A represents a group $-CHOH-CH_2$;

c) if appropriate, reacting a chlorinating agent with the compound (I) in which A represents $-CHOH-CH_2-$, in an inert solvent, in order to prepare the compound (I) in which A represents $-CHCl-CH_2-$;

d) if appropriate, carrying out a hydrogenation of the compound (I) in which A represents the acetylenic group $-C{\equiv}C-$, with nascent hydrogen, in order to prepare the compound (I) in which A represents the group $-CH=CH-$, in the form of a mixture of the cis and trans isomers, or carrying out a hydrogenation in the presence of a supported metal catalyst in order to prepare the ethylenic compound (I) in the cis form, or dehydrating the compound (I) in which A represents a group-$CHOH-CH_2-$ in order to prepare the ethylenic compound (I) in the trans form;

e) if appropriate, carrying out a hydrogenation of the compound (I) in which A represents a group -CH=CH- or a group -C≡C- in order to prepare the compound (I) according to the invention in which A represents the group $-CH_2-CH_2$; and

f) finally, if necessary, preparing an addition salt of a compound (I) by the addition of an appropriate mineral or organic acid.

6. A pharmaceutical composition in which at least one compound according to any one of claims 1, 2, 3 and 4 is present as the active principle.

7. A pharmaceutical composition according to claim 6 which is in the form of a dosage unit in which the active principle is mixed with a pharmaceutical excipient.

8. A pharmaceutical composition according to claim 6 or 7, characterized in that it contains from 0.5 to 1000 mg of active principle per dosage unit.

9. A pharmaceutical composition according to claim 7 or 8, characterized in that it contains 2.5 to 200 mg of active principle per dosage unit.

**Claims for the following Contracting State : ES**

1. A method of preparing a compound of the formula:

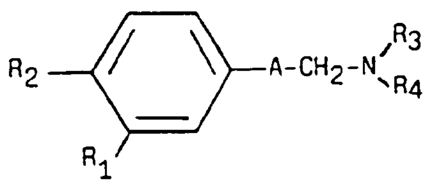

(I)

in which:

- $R_1$ represents a hydrogen atom or a halogen atom;
- $R_2$ represents a cyclohexyl or a phenyl;
- $R_3$ represents a cycloalkyl containing from 3 to 6 carbon atoms;
- $R_4$ represents a hydrogen atom, an alkyl containing from 1 to 6 carbon atoms or a cycloalkyl containing from 3 to 6 carbon atoms; and
- A represents a group selected from : $-CO-CH_2-$, $-CH(Cl)-CH_2-$, $-CH(OH)-CH_2-$, $-CH_2-CH_2-$, -CH=CH- and -C≡C-,

or one of its addition salts with mineral or organic acids, characterized in that it comprises:

a) carrying out a condensation reaction of formaldehyde and an amine of the formula $HNR_3R_4$, in which $R_3$ and $R_4$ are as defined above for (I), either with the acetophenone of the formula

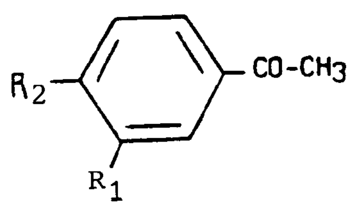

(II)

in which $R_1$ and $R_2$ are as defined above for (I), to give a compound (I) in which A represents the group $-CO-CH_2-$, or with a phenylacetylenic derivative of the formula

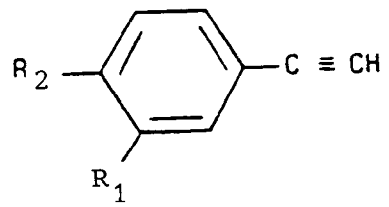

(III)

in which $R_1$ and $R_2$ are as defined above for (I), to give a compound (I) in which A represents the group -C≡C-;

b) if appropriate, reacting a reducing agent with the compound (I) in which A represents a group -CO-$CH_2$- in order to prepare the compound (I) in which A represents a group -CHOH-$CH_2$-;

c) if appropriate, reacting a chlorinating agent with the compound (I) in which A represents -CHOH-$CH_2$-, in an inert solvent, in order to prepare the compound (I) in which A represents -CHCl-$CH_2$-;

d) if appropriate, carrying out a hydrogenation of the compound (I) in which A represents the acetylenic group -C≡C-, with nascent hydrogen, in order to prepare the compound (I) in which A represents the group -CH=CH-, in the form of a mixture of the cis and trans isomers, or carrying out a hydrogenation in the presence of a supported metal catalyst in order to prepare the ethylenic compound (I) in the cis form, or dehydrating the compound (I) in which A represents a group -CHOH-$CH_2$- in order to prepare the ethylenic compound (I) in the trans form;

e) if appropriate, carrying out a hydrogenation of the compound (I) in which A represents a group -CH=CH- or a group -C≡C- in order to prepare the compound (I) according to the invention in which A represents the group -$CH_2$-$CH_2$-; and

f) finally, if necessary, preparing an addition salt of a compound (I) by the addition of an appropriate mineral or organic acid.

**2.** A method according to claim 1, characterized in that a pharmaceuticaly acceptable addition salt is prepared according to step f).

**3.** A method according to one of claims 1 or 2, characterized in that it comprises:

- carrying out a condensation reaction of formaldehyde and an amine of formula

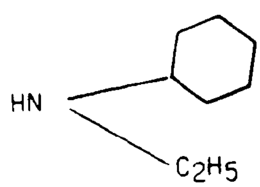

with the phenylacetylenic derivative of the formula

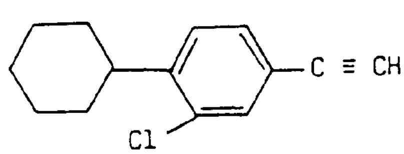

to obtain the compound of the formula:

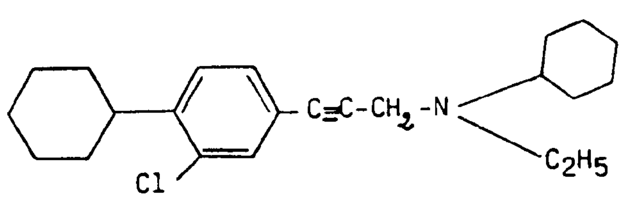

- carrying out a hydrogenation of said obtained compound in the presence of a supported metal catalyst to prepare the compound of the formula

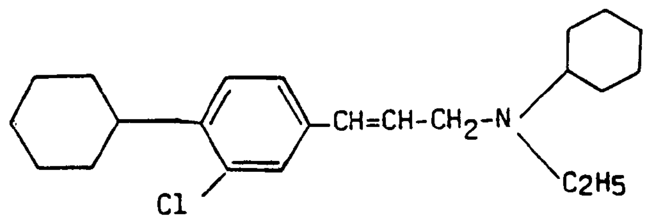

, cis.

isolated as base or as hydrochloride.

4. A method of preparing pharmaceutical compositions, characterized in that it consists in mixing at least one compound prepared according to any one of claims 1 to 3, to a pharmaceutical excipient.

5. A method according to claim 4, for the preparation of dosage units.

6. A method according to claim 5, for the preparation of dosage units containing from 0.5 to 1000 mg of active principle of formula (I).

7. A method according to claim 5, for the preparation of dosage units containing from 2.5 to 200 mg of active principle of formula (I).

**Claims for the following Contracting State : GR**

1. A compound of the formula

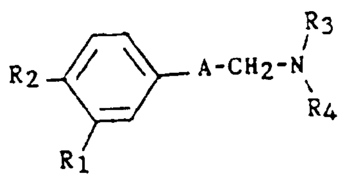

(I)

in which:
- $R_1$ represents a hydrogen atom or a halogen atom;
- $R_2$ represents a cyclohexyl or a phenyl;
- $R_3$ represents a cycloalkyl containing from 3 to 6 carbon atoms;
- $R_4$ represents a hydrogen atom, an alkyl containing from 1 to 6 carbon atoms or a cycloalkyl containing from 3 to 6 carbon atoms; and
- A represents a group selected from : $-CO-CH_2-$, $-CH(Cl)-CH_2-$, $-CH(OH)-CH_2-$, $-CH_2-CH_2-$, $-CH=CH-$ and $-C\equiv C-$,

or one of its addition salts with mineral or organic acids.

2. Pharmaceutically acceptable addition salts of a compound according to claim 1.

3. Cis-N-cyclohexyl-N-ethyl-3-(3-chloro-4-cyclohexyl-phenyl)prop-2-enylamine or one of its pharmaceutically acceptable addition salts.

4. Cis-N-cyclohexyl-N-ethyl-3-(3-chloro-4-cyclohexyl-phenyl)prop-2-enylamine hydrochloride.

5. A method of preparing the compounds (I) according to any one of claims 1 to 4, characterized in that it comprises:

a) carrying out a condensation reaction of formaldehyde and an amine of the formula $HNR_3R_4$, in which $R_3$ and $R_4$ are as defined in claim 1 for (I), either with the acetophenone of the formula

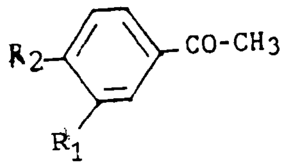

(II)

in which $R_1$ and $R_2$ are as defined in claim 1 for (I), to give a compound (I), in which A represents the group $-CO-CH_2-$, or with a phenylacetylenic derivative of formula

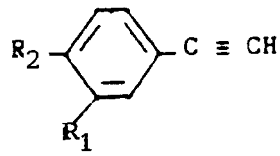

(III)

in which $R_1$ and $R_2$ are as defined in claim 1, to give a compound (I) in which A represents a group -C≡C-;
b) if appropriate, reacting a reducing agent with the compound (I) in which A represents a group $-CO-CH_2-$ in order to prepare the compound (I) in which A represents a group $-CHOH-CH_2$;
c) if appropriate, reacting a chlorinating agent with the compound (I) in which A represents $-CHOH-CH_2-$, in an inert solvent, in order to prepare the compound (I) in which A represents $-CHCl-CH_2-$;
d) if appropriate, carrying out a hydrogenation of the compound (I) in which A represents the acetylenic group -C≡C-, with nascent hydrogen, in order to prepare the compound (I) in which A represents the group -CH=CH-, in the form of a mixture of the cis and trans isomers, or carrying out a hydrogenation in the presence of a supported metal catalyst in order to prepare the ethylenic compound (I) in the cis form, or dehydrating the compound (I) in which A represents a group-$CHOH-CH_2-$ in order to prepare the ethylenic compound (I) in the trans form;
e) if appropriate, carrying out a hydrogenation of the compound (I) in which A represents a group -CH=CH- or a group -C≡C- in order to prepare the compound (I) according to the invention in which A represents the group $-CH_2-CH_2$; and
f) finally, if necessary, preparing an addition salt of a compound (I) by the addition of an appropriate mineral or organic acid.

**6.** A method of preparing pharmaceutical compositions, characterized in that it consists in mixing at least one compound according to any one of claims 1 to 4 to a pharmaceutical excipient.

**7.** A method according to claim 6, for the preparation of dosage units.

**8.** A method according to claim 7, for the preparation of dosage units containing from 0.5 to 1000 mg of active principle of formula (I)

**9.** A method according to claim 7, for the preparation of dosage units containing from 2.5 to 200 mg of active principle of formula (I).